# EUROPÄISCHE PATENTANMELDUNG

(11) **EP 2 127 607 A1**
(43) Veröffentlichungstag der Anmeldung: **02.12.2009**
(21) Anmeldenummer: 09006874.3
(22) Anmeldetag: 22.05.2009
(51) Int. Cl.: A61B 17/295

(54) **Medizinische Stanze**

(30) Priorität: 31.05.2008 DE 102008026353
(71) Anmelder: Karl Storz GmbH & Co. KG, 78532 Tuttlingen (DE)
(72) Erfinder: Oberländer, Martin, 78234 Engen (DE); Sauer, Michael, 78532 Tuttlingen (DE)
(74) Vertreter: Hofmeister, Frank

(57) **Zusammenfassung**

Die Erfindung betrifft eine medizinische Stanze mit einem Hohlschaft (2), einem am distale Ende des Hohlschaftes (2) angeordneten, aus einer starren Schneidkante (7) und einer gegenüber der starren Schneidkante (7) verschiebbaren Schneidkante (8) bestehenden Stanzwerkzeug (6) sowie mit einer am proximalen Ende des Hohlschaftes (2) angeordneten Handhabe (3), wobei die verschiebbare Schneidkante (8) und die Handhabe (3) über eine verschiebbar in dem Hohlschaft (2) gelagerte, als Schub-/Zugstange (9) ausgebildete Schub-/Zugeinrichtung (9) miteinander in Wirkverbindung stehen. Um eine medizinische Stanze (1) zu schaffen, die bei einfachem Aufbau gut zu reinigen ist, wird erfindungsgemäß vorgeschlagen, dass die Schub-/Zugstange (9) als hohles Rohr ausgebildet ist, die über das proximale Ende des Hohlschaftes (2) in den Hohlschaft (2) einsetzbar und aus dem Hohlschaft (2) herausziehbar ist.

## Beschreibung

Die Erfindung betrifft eine medizinische Stanze mit einem Hohlschaft, einem am distale Ende des Hohlschaftes angeordneten, aus einer starren Schneidkante und einer gegenüber der starren Schneidkante verschiebbaren Schneidkante bestehenden Stanzwerkzeug sowie mit einer am proximalen Ende des Hohlschaftes angeordneten Handhabe, wobei die verschiebbare Schneidkante und die Handhabe über eine verschiebbar in dem Hohlschaft gelagerte, als Schub-/Zugstange ausgebildete Schub-/Zugeinrichtung miteinander in Wirkverbindung stehen.

Bei den medizinischen Stanzen handelt es sich um Schneidwerkzeuge mit einer starren Schneidkante und einer gegenüber der starren Schneidkante verschiebbaren Schneidkante, wobei die beiden Schneidkanten nicht wie bei einer Schere oder dergleichen um einen Drehpunkt gegeneinander verschwenkbar sind, sondern in der Regel horizontal gegeneinander verfahrbar sind.

Bei den aus der Praxis bekannten medizinischen Stanzen besteht der als Schiebeschaft ausgebildete Instrumentenschaft aus einem starren Schaftteil und einem auf dem starren Schaftteil in einer Führung verschiebbar gelagerten verschiebbaren Schaftteil, wobei der verschiebbare Schaftteil über die Handhabe relativ zum starren Schaftteil verlagerbar ist. Diese Stanzen haben sich in der Praxis zwar durchaus bewährt, jedoch sind aufgrund der aufeinander gelagerten Schaftteile sehr kleine Schaftdurchmesser kaum herstellbar. Weiterhin sind dieses bekannten Stanzen zu Reinigungszwecken mit einigem Montageaufwand nur von distal zerlegbar, was die Handhabung bei der Reinigung deutlich erschwert.

Eine gattungsgemäße medizinische Stanze ist beispielsweise aus der EP 1 055 397 A1 bekannt.

Davon ausgehend liegt der Erfindung die **Aufgabe** zugrunde, eine medizinische Stanze der eingangs genannten Art so auszugestalten, dass diese bei einfachem Aufbau gut zu reinigen ist.

Die **Lösung** dieser Aufgabenstellung ist erfindungsgemäß **dadurch gekennzeichnet, dass** die Schub-/Zugstange als hohles Rohr ausgebildet ist, die über das proximale Ende des Hohlschaftes in den Hohlschaft einsetzbar und aus dem Hohlschaft herausziehbar ist.

Diese Ausgestaltung der Stanze als Hohlschaftinstrument mit einem hohlen Außenschaft und einer in dem Hohlschaft verschiebbar gelagerten Schub-/Zugstange hat den Vorteil, dass die Schub-/Zugstange zu Reinigungszwecken aus dem Hohlschaft entnehmbar ist, so dass einerseits der Hohlschaft separat, beispielsweise über einen integrierten Spülanschluss, reinigbar ist und andererseits die Schub-/Zugstange separat reinigbar ist.

Durch die erfindungsgemäße Ausbildung der Schub-/Zugstange als hohles Rohr, das über das proximale Ende des Hohlschaftes in den Hohlschaft einsetzbar und aus dem Hohlschaft herausziehbar ist, ist die Schub-/Zugstange einfach und gut zu reinigen, beispielsweise durch einfaches Durchspülen.

Das Koppeln der Schub-/Zugstange mit der Handhabe, insbesondere dem verschwenkbaren Griffteil der Handhabe, erfolgt erfindungsgemäß vorteilhafterweise mittels einer an der Handhabe oder an der Schub-/Zugstange gelagerte Überwurfmutter.

Um sicherzustellen, dass, insbesondere bei den winklig zur Schaftlängsachse ausgebildeten Schneidkanten des Stanzwerkzeugs, die mit der proximalseitig angeordneten verschiebbaren Schneidkante versehene Schub-/Zugstange immer richtig positioniert zur starren Schneidkante in den Hohlschaft eingesetzt wird, wird mit der Erfindung vorgeschlagen, dass an der Schub-/Zugstange und an der Handhabe miteinander korrespondierende Positionierungselemente ausgebildet sind, die ein Koppeln der beiden miteinander zu verbindenden Bauteile nur in der richtigen Position erlauben.

Weiterhin wird mit der Erfindung vorgeschlagen, dass die Positionierungselemente als an einem Bauteil ausgebildeter Stift und am anderen Bauteil ausgebildete Nut zur Aufnahme des Stiftes ausgebildet sind.

Gemäß einer praktischen Ausführungsform der Erfindung wird vorgeschlagen, dass die starre Schneidkante im Bereich des distalen Endes am Hohlschaft ausgebildet ist. Die Ausbildung der starren Schneidkante am Hohlschaft ermöglicht auf einfache Art und Weise die Ausbildung der einen Schneidkante des Stanzwerkzeugs. Zur Ausbildung der starren Schneidkante am Hohlschaft wird mit einer bevorzugten Ausführungsform der Erfindung vorgeschlagen, dass im Bereich des distalen Endes des Hohlschaftes eine radialwärts weisende Aussparung ausgebildet ist, deren nach proximal weisende Kante die starre Schneidkante bildet.

Zur Ausbildung der verschiebbaren Schneidkante des Stanzwerkzeugs wird erfindungsgemäß vorgeschlagen, dass die verschiebbare Schneidkante das distale Ende, der in dem Hohlschaft gelagerten Schub-/Zugstange bildet. Durch die Ausbildung der starren Schneidkante am Hohlschaft und der verschiebbaren Schneidkante am distalen Ende der verschiebbar im Hohlschaft gelagerten Schub-/Zugstange werden die beiden Schneidkanten des Stanzwerkzeugs auf konstruktiv besonders einfache Art ausgebildet.

Weiterhin wird mit einer ersten Ausführungsform der Erfindung vorgeschlagen, dass die starre Schneidkante und die verschiebbare Schneidkante parallel zueinander und im Wesentlichen rechtwinklig zur Längsachse des Schaftes ausgebildet sind.

Gemäß einer zweiten Ausführungsform der Erfindung wird vorgeschlagen, dass die starre Schneidkante und die verschiebbare Schneidkante ebenfalls parallel zueinander, jedoch in einem vom rechten Winkel abweichenden Winkel, vorzugsweise von 45°, zur Längsachse des Schaftes ausgebildet sind.

Schließlich wird mit der Erfindung vorgeschlagen, dass die Handhabe aus einem starren Griffteil und einem gegenüber dem starren Griffteil verschwenkbaren Griffteil besteht, wobei die in dem Hohlschaft gelagerte Schub-/Zugstange mit dem verschwenkbaren Griffteil gekoppelt ist. Die Ausbildung der Handhabe mit den beiden Griffteilen ermöglicht ein sicheres Handhaben der Stanze und dosiertes Verschieben der mit dem verschwenkbaren Griffteil gekoppelten Schub-/Zugstange innerhalb des Hohlschaftes, was seinerseits ein dosiertes und präzises Betätigen des Stanzwerkzeugs ermöglicht.

Weitere Merkmale und Vorteile der Erfindung ergeben sich anhand der zugehörigen Zeichnung, in der zwei Ausführungsbeispiele einer erfindungsgemäßen medizinischen Stanze nur beispielhaft dargestellt sind, ohne die Erfindung auf diese Ausführungsbeispiele zu beschränken. In der Zeichnung zeigt:
- Fig. 1: eine teilweise geschnittene Seitenansicht einer ersten Ausführungsform einer erfindungsgemäßen medizinischen Stanze;
- Fig. 2: eine vergrößerte Ansicht des Details II gemäß Fig. 1;
- Fig. 3: eine Ansicht gemäß Fig. 2, jedoch eine zweite Ausführungsform darstellend;
- Fig. 4: eine perspektivische Ansicht der Stanze gemäß Fig. 1, jedoch im zerlegten Zustand;
- Fig. 5: eine perspektivische Ansicht gemäß Fig. 4, jedoch die Stanze im zusammengesetzten Zustand darstellend;
- Fig. 6: eine vergrößerte Ansicht des Details VI gemäß Fig. 5
- Fig. 7: eine Seitenansicht der Detailansicht gemäß Fig. 6;
- Fig. 8: eine vergrößerte Ansicht des Details VIII gemäß Fig. 5 und
- Fig. 9: eine Seitenansicht der Detailansicht gemäß Fig. 8.

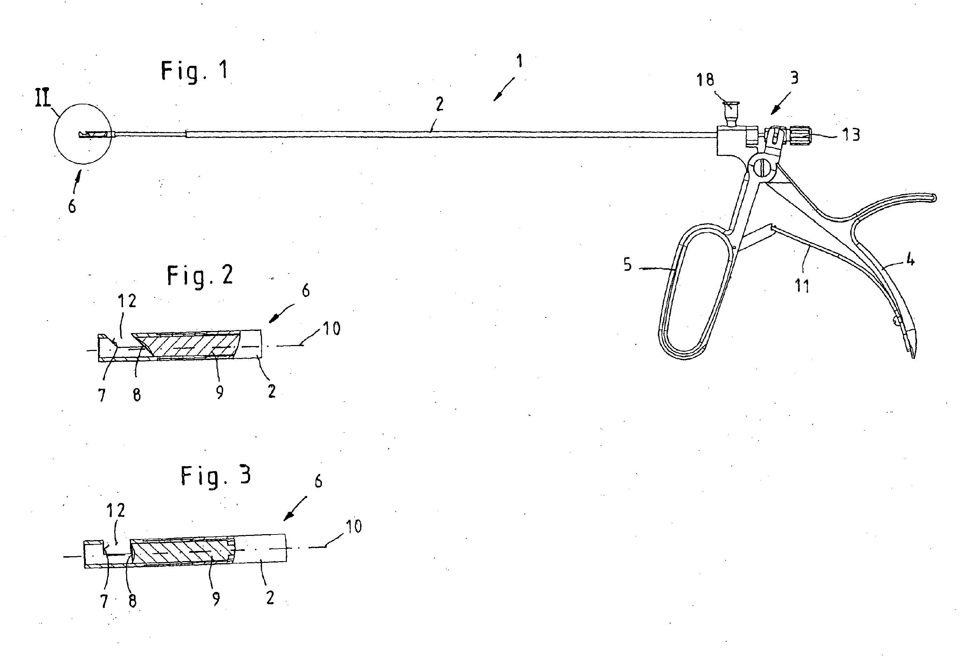

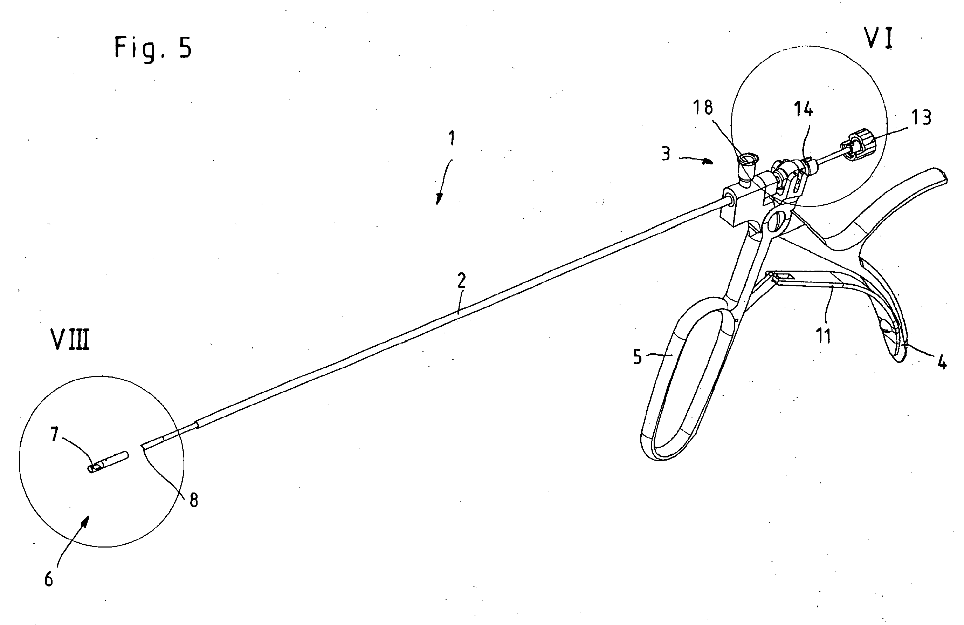

Die in den Abbildungen Fig. 1, 3, 4 und 5 vollständig dargestellte medizinische Stanze 1 besteht im Wesentlichen aus einem als Hohlschaft ausgebildeten Schaft 2, an dessen proximalem Ende eine Handhabe 3 angeordnet ist, die aus einem starren Griffteil 4 und einem gegenüber dem starren Griffteil 4 verschwenkbaren Griffteil 5 besteht. Am distalen Ende des Schaftes 2 ist ein Stanzwerkzeug 6 angeordnet, das aus einer starren Schneidkante 7 und einer gegenüber der starren Schneidkante 7 verschiebbaren Schneidkante 8 besteht, wobei die verschiebbare Schneidkante 8 am distalen Ende einer verschiebbar im Hohlschaft 2 gelagerten hohlen Schub-/Zugstange 9 angeordnet ist.

Wie aus Fig. 1 und 2 ersichtlich, stehen die verschiebbare Schneidkante 8 des Stanzwerkzeugs 6 und der verschwenkbare Griffteil 5 der Handhabe 3 über die im Hohlschaft 2 gelagerte, als Schub-/Zugstange 9 ausgebildete Schub-/Zugeinrichtung derart in Wirkverbindung miteinander, dass durch das Verstellen des verschwenkbaren Handgriffs 5 der Handhabe 3 die verschiebbare Schneidkante 8 über die Verlagerung des Schub-/Zugstange 9 in Richtung der Längsachse 10 des Schaftes 2 auf die starre Schneidkante 7 des Stanzwerkzeugs 6 zu bzw. umgekehrt von der starren Schneidkante 7 fort verschiebbar ist.

Bei der dargestellten Ausführungsform bewirkt das Zusammendrücken der Griffteile 4 und 5 der Handhabe 3 eine Verlagerung der Schub-/Zugstange 9 in distale Richtung und somit ein Verschieben der verschiebbaren Schneidkante 8 in Richtung der starren Schneidkante 7. Zwischen den beiden Griffteilen 4 und 5 der Handhabe 3 ist ein Federelement 11 angeordnet, welches die Griffteile 4 und 5 auseinanderdrückt und somit die Schub-/Zugstange 9 in proximale Richtung vorspannt, in der die verschiebbare Schneidkante 8 und die starre Schneidkante 7 am weitesten voneinander entfernt sind und die in Fig. 2 und 3 dargestellten Positionen zueinander einnehmen.

Der Aufbau und die Anordnung der Schneidkanten 7 und 8 des Stanzwerkzeugs 6 ist insbesondere den Abbildungen Fig. 2 und 3 zu entnehmen, die zwei Ausführungsbeispiele zur Ausgestaltung der Schneidkanten 7 und 8 zeigen. In beiden Fällen bildet die verschiebbare Schneidkante 8 das distale Ende der im Hohlschaft 2 verschiebbar gelagerten hohlen Schub-/Zugstange 9.

Zur Ausbildung der starren Schneidkante 7 ist im Bereich des distalen Endes des Hohlschaftes 2 eine radialwärts weisende Aussparung 12 im Hohlschaft 2 ausgebildet, deren nach proximal weisende Kante die starre Schneidkante 7 bildet.

Die beiden dargestellten Ausführungsformen zur Ausgestaltung der starren und verschiebbaren Schneidkanten 7 und 8 unterscheiden sich voneinander durch die Ausrichtung der Schneidkanten 7 und 8 zur Längsachse 10 des Schaftes 2. Zueinander sind die Schneidkanten 7 und 8 bei beiden Ausführungsformen parallel ausgerichtet

Wie aus Fig. 1 und 2 ersichtlich, sind die starre Schneidkante 7 und die verschiebbare Schneidkante 8 bei der ersten Ausführungsform parallel zueinander und in einem vom rechten Winkel abweichenden Winkel, vorzugsweise einem Winkel von 45°, zur Längsachse 10 des Schaftes 2 ausgebildet. Bei der in Fig. 3 dargestellten zweiten Ausführungsform sind die starre Schneidkante 7 und die verschiebbare Schneidkante 8 im Wesentlichen rechtwinklig zur Längsachse 10 des Schaftes 2 ausgebildet.

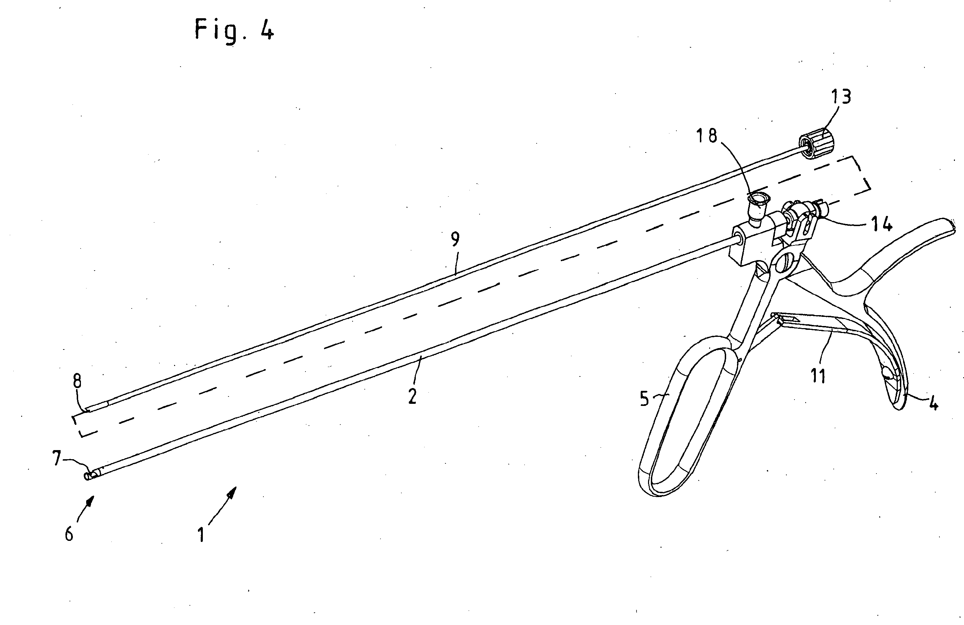

Das Montieren und Demontieren einer solchermaßen ausgebildeten medizinischen Stanze 1 wird nachfolgend insbesondere anhand der Abbildungen Fig. 4 bis 9 beschrieben.

Zur Montage der medizinischen Stanze 1 wird ausgehend von dem in Fig. 4 dargelegten zerlegten Zustand zunächst die hohle Schub-/Zugstange 9 vom proximalen Ende her in den Hohlschaft 2 eingeschoben und dann mit dem verschwenkbaren Griffteil 5 der Handhabe 3 derart gekoppelt, dass eine Betätigung des verschwenkbaren Griffteils 5 eine Längsverlagerung der Schub-/Zugstange 9 im Hohlschaft 2 bewirkt.

Bei der dargestellten Ausführungsform erfolgt das Festlegen der Schub-/Zugstange 9 am verschwenkbaren Griffteil 5 der Handhabe 3 über eine unverlierbar an der Schub-/Zugstange 9 gelagerte Überwurfmutter 13, die auf einen entsprechenden Gewindebereich 14 der Handhabe 3 aufgeschraubt wird. Selbstverständlich ist es auch möglich, die Überwurfmutter 13 an der Handhabe 3 zu lagern und den Gewindebereich am proximalen Ende der Schub-/Zugstange 9 auszubilden.

Insbesondere bei der Ausbildung der Schneidkanten 7 und 8 des Stanzwerkzeugs 6 mit einem vom rechten Winkel abweichenden Winkel zur Längsachse 10 des Schaftes 2 ist es zwingend erforderlich, dass die die verschiebbare Schneidkante 8 tragende Schub-/Zugstange 9 in der richtigen Ausrichtung zur am Hohlschaft 2 ausgebildeten starren Schneidkante 7 in den Hohlschaft 2 eingeführt wird. Um ein lagerichtiges und richtig positioniertes Einführen der Schub-/Zugstange 9 in den Hohlschaft 2 zu gewährleisten, sind an der Schub-/Zugstange 9 und an der Handhabe 3 miteinander korrespondierende Positionierungselemente 15 ausgebildet, die eine Fehlpositionierung verhindern.

Bei der dargestellten Ausführungsform sind die Positionierungselemente 15 als an der Schub-/Zugstange 9 ausgebildeter Stift 16 und an der Handhabe 3 ausgebildete Nut 17 zur Aufnahme des Stiftes 16 ausgebildet. Erst wenn der Stift 16 vollständig in die Nut 17 eingeschoben wurde, lässt sich die Überwurfmutter 13 zum Festlegen der Schub-/Zugstange 9 an der Handhabe 3 auf den Gewindebereich 14 aufschrauben. Selbstverständlich ist es auch möglich, die Ausbildung des Stiftes 16 und der Nut 17 am jeweils anderen Bauteil 3 und 9 vorzunehmen.

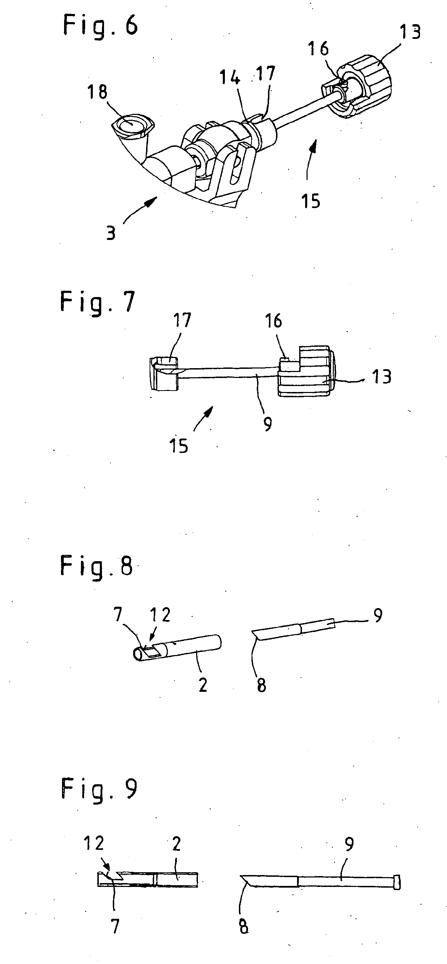

Die durch die Positionierungselemente 15 bewirkte lagerichtige Ausrichtung der Schneidkanten 7 und 8 des Stanzwerkzeugs 6 zueinander ist insbesondere den Abbildungen Fig. 6 bis Fig. 9 zu entnehmen.

Nach dem lagerichtigen Einführen der Schub-/Zugstange 9 in den Hohlschaft 9 und dem Festlegen der Schub-/Zugstange 9 am verschwenkbaren Griffteil 5 der Handhabe 3 mittels der Überwurfmutter 13 ist die medizinische Stanze 1 einsatzbereit.

Die Demontage der medizinischen Stanze 1, beispielsweise zu Reinigungszwecken, erfolgt in umgekehrter Reihenfolge und wird durch das Abschrauben der Überwurfmutter 13 vom Gewindebereich 14 und somit das Lösen der Kopplung zwischen der Schub-/Zugstange 9 und der Handhabe 3 eingeleitet.

Nachfolgend kann die Schub-/Zugstange über das proximale Ende des Schaftes 2 aus dem Hohlschaft 2 herausgezogen werden. Das Reinigen des Hohlschaftes 2 erfolgt beispielsweise über einen an der Handhabe 3 angeordneten Spülanschluss 18. Die aus dem Hohlschaft 2 herausgezogene Schub-/Zugstange 9 lässt sich als separates Bauteil ebenfalls einfach und gut reinigen.

Eine solchermaßen ausgebildete medizinische Stanze 1 zeichnet sich **dadurch aus, dass** sie bei einfachem konstruktiven Aufbau einerseits die Herstellung sehr kleiner Schaftdurchmesser ermöglicht und andererseits gut zu reinigen ist.

### Bezugszeichenliste

- 1: medizinische Stanze
- 2: Schaft/Hohlschaft
- 3: Handhabe
- 4: starrer Griffteil
- 5: verschwenkbarer Griffteil
- 6: Stanzwerkzeug
- 7: starre Schneidkante
- 8: verschiebbare Schneidkante
- 9: Schub-/Zugstange
- 10: Längsachse
- 11: Federelement
- 12: Aussparung
- 13: Überwurfmutter
- 14: Gewindebereich
- 15: Positionierungselemente
- 16: Stift
- 17: Nut

- 18: Spülanschluss

## Patentansprüche

1. Medizinische Stanze mit einem Hohlschaft (2), einem am distale Ende des Hohlschaftes (2) angeordneten, aus einer starren Schneidkante (7) und einer gegenüber der starren Schneidkante (7) verschiebbaren Schneidkante (8) bestehenden Stanzwerkzeug (6) sowie mit einer am proximalen Ende des Hohlschaftes (2) angeordneten Handhabe (3), wobei die verschiebbare Schneidkante (8) und die Handhabe (3) über eine verschiebbar in dem Hohlschaft (2) gelagerte, als Schub-/Zugstange (9) ausgebildete Schub-/Zugeinrichtung (9) miteinander in Wirkverbindung stehen,
**dadurch gekennzeichnet,**
**dass** die Schub-/Zugstange (9) als hohles Rohr ausgebildet ist, die über das proximale Ende des Hohlschaftes (2) in den Hohlschaft (2) einsetzbar und aus dem Hohlschaft (2) herausziehbar ist.

2. Medizinische Stanze nach Anspruch 1, **dadurch gekennzeichnet, dass** die Schub-/Zugstange (9) über eine Überwurfmutter (13) an der Handhabe (3) festlegbar ist.

3. Medizinische Stanze nach Anspruch 1 oder 2, **dadurch gekennzeichnet, dass** an der Schub-/Zugstange (9) und an der Handhabe (3) miteinander korrespondierende Positionierungselemente (15) ausgebildet sind.

4. Medizinische Stanze nach Anspruch 3, **dadurch gekennzeichnet, dass** die Positionierungselemente (15) als an einem Bauteil (3 oder 9) ausgebildeter Stift (16) und am anderen Bauteil (9 oder 3) ausgebildete Nut (17) zur Aufnahme des Stiftes (16) ausgebildet sind.

5. Medizinische Stanze nach einem der Ansprüche 1 bis 4" **dadurch gekennzeichnet, dass** die starre Schneidkante (7) im Bereich des distalen Endes am Hohlschaft (2) ausgebildet ist.

6. Medizinische Stanze nach Anspruch 5, **dadurch gekennzeichnet, dass** im Bereich des distalen Endes des Hohlschaftes (2) eine radialwärts weisende Aussparung (12) ausgebildet ist, deren nach proximal weisende Kante die starre Schneidkante (7) bildet.

7. Medizinische Stanze nach einem der Ansprüche 1 bis 6, **dadurch gekennzeichnet, dass** die verschiebbare Schneidkante (8) das distale Ende der in dem Hohlschaft (2) gelagerten Schub-/Zugstange (9) bildet.

8. Medizinische Stanze nach einem der Ansprüche 1 bis 7, **dadurch gekennzeichnet, dass** die starre Schneidkante (7) und die verschiebbare Schneidkante (8) parallel zueinander und im Wesentlichen rechtwinklig zur Längsachse (10) des Schaftes (2) ausgebildet sind.

9. Medizinische Stanze nach einem der Ansprüche 1 bis 7, **dadurch gekennzeichnet, dass** die starre Schneidkante (7) und die verschiebbare Schneidkante (8) parallel zueinander und in einem vom rechten Winkel abweichenden Winkel, vorzugsweise von 45°, zur Längsachse (10) des Schaftes (2) ausgebildet sind.

10. Medizinische Stanze nach einem der Ansprüche 1 bis 9, **dadurch gekennzeichnet, dass** die Handhabe (3) aus einem starren Griffteil (4) und einem gegenüber dem starren Griffteil (4) verschwenkbaren Griffteil (5) besteht, wobei die in dem Hohlschaft gelagerte Schub-/Zugstange (9) mit dem verschwenkbaren Griffteil (5) gekoppelt ist.
